# FASCICULE DE BREVET EUROPEEN

(11) **EP 1 382 355 B1**
(45) Date de publication et mention de la délivrance du brevet: **23.08.2006**
(21) Numéro de dépôt: 03291703.1
(22) Date de dépôt: 09.07.2003
(51) Int. Cl.: A61L 9/14, A61L 2/18, A61L 2/22

(54) **Procédé de désinfection de locaux d'élevage**
Verfahren zur Desinfektion von Tierställen
Process for the disinfection of livestock barns

(30) Priorité: 11.07.2002 FR 0208733
(43) Date de publication de la demande: 21.01.2004
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Le Rouzic, Daniel, 95120 Ermont (FR); Gamet, Jean-Claude, 71460 Saint Martin du Tartre (FR)
(74) Mandataire: Conan, Philippe Claude

(56) Documents cités:
- EP-A- 0 475 505
- EP-A- 1 226 835
- WO-A-90/12600
- WO-A-99/66961
- FR-A- 2 772 620
- GB-A- 1 570 492

## Description

L'invention se rapporte à un nouveau procédé de désinfection de locaux et à la composition mise en oeuvre.

La bio-sécurité des produits alimentaires, implique une prise de responsabilité plus importante de la filière alimentaire et plus particulièrement des éleveurs quant à la qualité et à l'innocuité des viandes mises à la consommation humaine.

Il en découle l'obligation impérieuse d'élever les animaux et de produire la viande dans d'excellentes conditions sanitaires et aussi d'être capable d'en apporter la preuve.

Les conditions d'hygiène dans lesquelles opère la filière viande sont donc essentielles pour atteindre cet objectif.

C'est pourquoi, les procédures de nettoyage et de désinfection des locaux d'élevages s'inscrivent dans la stratégie globale de l'hygiène de cette filière. Ces procédures comprennent notamment les étapes suivantes :
(a) - la préparation des bâtiments comme le dégagement de tous leurs accès ou la vidange des pré-fosses,
(b) - le nettoyage des surfaces, à l'eau chaude avec un nettoyant-dégraissant,
(c) - la désinfection primaire des surfaces, avec un désinfectant à large spectre,
(d) - la désinfection secondaire ou terminale des surfaces, par sublimation de formol ou aérolisation d'un désinfectant et,
(e) - le contrôle bactériologique.

Ces procédures permettent ainsi d'assainir les surfaces tout en quantifiant les résultats obtenus à l'issue de chaque étape. Généralement, le nettoyage diminue de 10 fois la population bactérienne présente en fin de bande, la désinfection primaire réduit de 10³ le nombre de germes restant après le nettoyage et la désinfection secondaire réduit de 10 fois le nombre de germes survivants.

Actuellement, la désinfection primaire des surfaces (sols et parois) est effectuée à l'aide d'un canon à mousse en utilisant des désinfectants à large spectre. Ces désinfectants sont généralement des associations de différents principes actifs, bactéricides, fongicides et virucides comprenant des aldéhydes comme le glutaraldéhyde. Ces derniers, outre leur toxicité élevée, polymérisent sur les surfaces et conduisent progressivement à la formation d'un film gras non hygiénique. De plus, les aldéhydes sont des fixateurs de protéines. De ce fait, toute salissure de cette nature, non éliminée dans l'étape préalable de nettoyage, peut être fortement fixée.

Quant à la désinfection secondaire, trois techniques sont retenues :
(a) - La sublimation du formol qui est un désinfectant très actif mais d'un emploi très délicat. Les locaux doivent être totalement hermétiques et le traitement doit durer 12 heures. De plus le délai de récupération est trop long car il faut aérer longtemps les locaux pour atteindre la valeur limite d'exposition aux vapeurs (VLE) ;
(b) - L'aérolisation d'un désinfectant qui consiste en la production d'un brouillard de fines particules de désinfectant de taille de l'ordre de 10 à 30 microns. Cette méthode est difficile à mettre en oeuvre pour obtenir un brouillard stable et homogène dans tout le local. Les produits désinfectants nécessitent trois à quatre heures de contacts ;
(c) - La thermonébulisation d'un désinfectant consistant en la production d'un brouillard de très fines particules de désinfectant de taille de l'ordre de 0,5 à 1 micron. On obtient par cette méthode une dispersion rapide et homogène du désinfectant. Cette dernière méthode est devenue le procédé de référence en matière de désinfection secondaire.

Cependant, lorsque cette méthode est mise en oeuvre avec des désinfectants aldéhydiques tels que le glutaraldéhyde, le temps de contact nécessaire est toujours supérieur à deux heures et il se produit une polymérisation du composé, qui conduit peu à peu à l'encrassement du thermonébulisateur. D'autre part comme, pour les méthodes précédentes, les locaux doivent rester inoccupés pendant 12 heures pour atteindre la valeur limite d'exposition aux vapeurs (VLE). De plus, au vu des composés utilisés, il est impossible de combiner le nettoyage et la désinfection en une seule étape.

La demande de brevet européen EP 1 226 835, faisant partie de l'état de la technique au sens de l'article 54(3) CBE divulgue un procédé de désinfection de locaux comprenant une étape de thermonébulisation d'une solution aqueuse d'acide peracétique.

La demande de brevet français FR 2 772 620, divulgue une composition désinfectante et fongicide, à base d'acide peracétique et d'oxyde d'amine, pouvant être utilisée pour désinfecter les surfaces dures.

La demande internationale WO 99/12600 divulgue une méthode de désinfection d'un local par atomisation d'un désinfectant aqueux.

La demande internationale WO 99/66961 divulgue une méthode de désinfection d'un local par nébulisation d'un désinfectant aqueux comme l'acide peracétique avec activation avec des ultrasons.

Le brevet britannique GB 1 570 492 divulgue une méthode de stérilisation d'un objet par aspersion d'une solution aqueuse d'acide peracétique.

La demande de brevet européen EP 0 475 505 divulgue une méthode de désinfection d'une hotte à flux laminaire par nébulisation d'acide peracétique.

La demanderesse a donc cherché à mettre au point un nouveau procédé de désinfection par thermonébulisation, qui n'ait pas les inconvénients exposés ci-dessus.

C'est pourquoi, l'invention a pour objet un procédé de nettoyage et de désinfection d'un local, comprenant au moins une étape de désinfection primaire, suivie d'au moins une étape de désinfection secondaire réalisée par thermonébulisation d'une solution aqueuse d'acide peracétique, caractérisé en ce que l'étape de désinfection primaire est effectuée par pulvérisation d'un mélange d'une solution aqueuse d'acide peracétique avec au moins un agent moussant..

L'agent tensioactif moussant utilisé dans le procédé décrit ci-dessus, est un composé résistant suffisamment à l'oxydation par l'acide peracétique et/ou par le peroxyde d'hydrogène.

Comme exemples de tensioactif moussant il y a les oxydes d'amine de formule (I) :

(R₁)(R₂)(R₃)N→O (I)

dans laquelle R₁ représente un radical aliphatique linéaire ou ramifié, saturé ou insaturé comprenant de 6 à 30 atomes de carbone et plus particulièrement de 8 à 18 atomes de carbone, et R₂ et R₃, identiques ou différents, représentent indépendamment l'un de l'autre un radical alkyle linéaire ou ramifié, comportant de 1 à 4 atomes de carbone ou un radical hydroxyalkyle, linéaire ou ramifié, comportant de 1 à 4 atomes de carbone.

Comme composé de formule (I) approprié à la présente invention, il y a plus particulièrement l'oxyde de cocodiméthylamine, l'oxyde de myristamine, l'oxyde de di(hydroxyéthyl) cocoamine, l'oxyde de diméthyl stéarylamine ou l'oxyde de diéthyl stéarylamine, comme les composés commercialisés sous le nom AROMOX™ et notamment l'AROMOX™ MCD-W.

La pulvérisation du mélange de la solution aqueuse d'acide peracétique avec l'agent moussant est de préférence réalisée au moyen d'un canon à mousse.

Le procédé est mis en oeuvre de façon à pulvériser une quantité d'acide peracétique comprise entre 0,5 g et 2 g par mètre carré de surface traitée et une quantité d'agent moussant comprise entre 1,5 g et 3 g par mètre carré de surface traitée.

La solution aqueuse d'acide peracétique est utilisée soit sans être préalablement diluée soit à un taux dilution généralement compris entre 0,05 et 0,1. On y ajoute l'agent moussant en une quantité telle que le rapport pondéral acide peracétique / agent moussant dans la composition finale à pulvériser, soit compris entre 0,5 et 2, avant mise en oeuvre du canon à mousse. On peut ainsi utiliser des solutions plus ou moins concentrées en acide peracétique.

Généralement la concentration en acide peracétique n'excède pas 20 % en poids et sera supérieure ou égale à 1 % en poids ; la concentration en acide acétique est comprise entre 2 % et 45 % en poids, et la concentration en peroxyde d'hydrogène est comprise entre 2 % et 35 % en poids.

L'invention a plus particulièrement pour objet, un procédé tel que défini ci-dessus, dans lequel on utilise une solution aqueuse d'acide peracétique comprenant :
- entre 1 % et 10% en poids d'acide peracétique,
- entre 5% et 30% et plus particulièrement entre 5% et 25 % en poids, de peroxyde d'hydrogène et,
- entre 5 % et 25 % et plus particulièrement entre 5% et 15% en poids, d'acide acétique,
que l'on dilue dans l'eau à un taux compris entre 0,05 et 0,1 pour donner une solution diluée à laquelle on ajoute un agent moussant en une quantité telle que le rapport pondéral acide peracétique / agent moussant dans la composition finale soit compris entre 0,75 et 1,5.

La solution aqueuse d'acide peracétique mise en oeuvre dans le procédé objet de la présente invention, peut aussi comprendre un ou plusieurs composés auxiliaires choisis parmi de 0,0001 % à 3 % en poids d'oxyde d'amine, de 0,001 % à 3 % en poids d'agents tensioactifs non ioniques, 0,001 % à 2 % d'inhibiteurs de corrosion, de 0,001 % à 3 % en poids d'agents stabilisants et de 0,001 % en poids à 1 % en poids de catalyseurs acides.

Comme oxyde d'amine, on préfère les composés de formule (I), telle que définie précédemment.

Comme agent tensioactif non-ionique approprié à la présente invention, il y a notamment les composés commerciaux suivants :

| nom | composition chimique |
|---|---|
| Génapol™ 2822 | mélange d'alcools alkoxylés en C₁₀, C₁₂, C₁₄, (5 OE, 4 OP) |
| Génapol™ 2908D | mélange d'alcools alkoxylés en C₁₁, C₁₃, C₁₅, (6 à 7 OE, 3 OP) |
| Génapol™ 2909 | mélange d'alcools alkoxylés en C₁₂ et C₁₄, (5 OE, 3 OP) |
| Simulsol™ NW 900 | mélange d'alcools alkoxylés (x OE, y OP) |
| Triton™ DF 12 | Ether benzylique d'alcools alkoxylés en C₈, C₁₀, (2 OE, 5 OP) |
| Triton™ CF 10 | Ether benzylique d'alcools éthoxylés en C₈, (16 OE) |
| Triton™ DF 16 | mélange d'alcools alkoxylés en C₈, C₁₀, (6 OE, 3 OP) |
| Akypo™ RO 90 | Ether carboxylique éthoxylé en C₁₆, C₁₈ (9 OE) |
| Dowfax™ 20b102 | |
| Akypo™ LF 2 | Ether carboxylique éthoxylé en C₈ (8 OE) |
| Akypo™ LF 4 | Ether carboxylique éthoxylé en C₆, C₈ (7 OE) |

On préfère les composés de formule (II) :

R₄-O-[CH(R₅)-CH(R₆)]ₙ-OR₇ (II)

dans laquelle R₄ représente un radical aliphatique linéaire ou ramifié, saturé ou insaturé comprenant de 6 à 30 atomes, et plus particulièrement de 10 à 18 atomes de carbone, et R₅ et R₆, identiques ou différents, représentent indépendamment l'un de l'autre un atome d'hydrogène ou un radical méthyle, R₇ représente un atome d'hydrogène, un radical alkyle linéaire ou ramifié, comportant de 1 à 4 atomes ou un radical benzyle et représente un nombre compris entre 1 et 50 et est de préférence inférieur à 20. Dans la définition de la formule (II), le groupe -[CH(R₅)-CH(R₆)]ₙ- peut représenter, soit une chaîne polyéthoxylée (R₅ et R₆ = H] soit une chaîne polypropoxylée (l'un des R₅ ou R₆ = H et l'autre est un radical méthyle), soit une chaîne polybutoxylée (R₅ et R₆ = CH₃], soit une chaînes constituée d'un mélange de deux sortes ou des trois sortes de groupes, éthoxyle, propoxyles ou butoxyles, les différents fragments étant distribués au sein de ladite chaîne, de manière séquencée ou aléatoire.

Les inhibiteurs de corrosion, les agents stabilisants et les catalyseurs acides sont ceux habituellement présents dans les solutions aqueuses d'acide peracétique.

L'invention a plus particulièrement pour objet un procédé tel que défini précédemment, dans lequel on utilise une solution aqueuse d'acide peracétique comprenant :
- entre 3 % et 6 % en poids d'acide peracétique,
- entre 10 % et 20 % en poids de peroxyde d'hydrogène et.
- entre 10 % et 20 % en poids d'acide acétique,
que l'on dilue dans l'eau à un taux égal à 0,075, pour donner une solution diluée à laquelle on ajoute un agent moussant en une quantité telle que le rapport pondéral acide peracétique / agent moussant dans la composition finale à pulvériser soit égal à 1.

Les solutions mises en oeuvre dans les aspects particuliers du procédé précédent contiennent aussi éventuellement :
entre 0,1 % et 0,5 % en poids d'agent stabilisant,
entre 0,5 % et 1 % d'inhibiteur de corrosion et
entre 0 % et 1 % d'acide fort.

Dans le procédé tel que défini précédemment, l'étape de thermonébulisation est mise en oeuvre de façon à traiter le local avec une quantité d'acide peracétique comprise entre 50 ppm et 300 ppm par mètre cube de local à désinfecter et de préférence entre 50 ppm et 100 ppm par mètre cube de local à désinfecter.

La température de thermonébulisation est généralement comprise entre 0°C et 45°C et est de préférence comprise entre 25°C et 35°C.

La solution est utilisée soit sans être préalablement diluée, soit à un taux dilution généralement compris entre 1 et 1/10. La dilution est effectuée manuellement avant mise en oeuvre de l'appareil à thermonébuliser. On utilise des solutions plus ou moins concentrées en acide peracétique.

Généralement la concentration en acide peracétique n'excède pas 20 % en poids et sera supérieure ou égale à 1 % en poids ; la concentration en acide acétique est comprise entre 2 % et 45 %, et la concentration en peroxyde d'hydrogène est comprise entre 2 % et 35 % en poids.

Dans le procédé tel que défini ci-dessus, on peut utiliser lors de l'étape de thermonébulisation, une solution aqueuse d'acide peracétique comprenant :
- entre 1% et 10 % en poids d'acide peracétique,
- entre 5 % et 30 % et plus particulièrement entre 5 % et 25 % en poids, de peroxyde d'hydrogène et,
- entre 5 % et 25 % et plus particulièrement entre 5% et 15% en poids, d'acide acétique, d'acide acétique,
qui est diluée dans l'eau à un taux compris entre 1 / 2 et 1 / 5 puis thermonébulisée.

La solution aqueuse d'acide peracétique mise en oeuvre dans cette étape du procédé objet de la présente invention, peut aussi comprendre un ou plusieurs composés auxiliaires choisis parmi de 0,0001 % à 3 % en poids d'oxyde d'amine, de 0,001 % à 3 % en poids d'agents tensioactifs non ioniques, 0,001 % à 2 % d'inhibiteurs de corrosion, de 0,001 % à 3 % en poids d'agents stabilisants et de 0,001 % en poids à 1 % en poids de catalyseurs acides.

Comme oxyde d'amine, on préfère les composés de formule (I), telle que définie précédemment.

Comme agent tensioactif non-ionique approprié à la présente invention, il y a ceux définis précédemment pour la solution utilisée dans l'étape de désinfection primaire.

Dans le procédé tel que défini ci-dessus, on peut utiliser lors de l'étape de thermonébulisation, une solution aqueuse d'acide peracétique comprenant :
- entre 3 % et 6 % en poids d'acide peracétique,
- entre 10 % et 20 % en poids de peroxyde d'hydrogène et.
- entre 10 % et 20 % en poids d'acide acétique,
qui est diluée au tiers avec de l'eau puis thermonébulisée.

Les solutions mises en oeuvre dans les variantes particulières précédentes contiennent aussi éventuellement
- entre 0,1 % et 0,5 % en poids d'agent stabilisant,
- entre 0,5 % et 1 % d'inhibiteur de corrosion et
- entre 0 % et 1 % d'acide fort.

Le procédé tel que défini précédemment comprend éventuellement une étape de lavage, préalable à l'étape de désinfection primaire.

Par étape de lavage, on entend dans le cadre de la présente invention, étape de lavage à l'eau chaude ou avec une solution aqueuse d'un ou plusieurs détergents, suivie si désiré ou si nécessaire, d'une ou plusieurs étapes de rinçage à l'eau chaude ou à l'eau froide.

L'invention a de préférence pour objet un procédé tel que défini précédemment dans lequel l'étape de lavage et l'étape de désinfection primaire constitue une seule étape de lavage et de désinfection effectuée par pulvérisation d'un mélange d'une solution aqueuse d'acide peracétique avec au moins un agent moussant et détergent.

Selon cette option préférée, le procédé tel que défini précédemment peut comporter une étape de rinçage à l'eau, préalablement à l'étape de thermonébulisation.

L'agent tensioactif détergent et moussant utilisé dans le procédé décrit ci-dessus, est un composé résistant à l'oxydation par l'acide peracétique et/ou par le peroxyde d'hydrogène.
Comme agent moussant et détergent il y a par exemple :
- les tensioactifs anioniques, tels que par exemple les alkylbenzènesulfonates, paraffine et oléfine sulfonates, les diphényl oxyde disulfonates, les alkyl sulfates, alkyl ether sulfates, alkyl phosphates, alkyl ether phosphates ou dialkylsulfosuccinates ;
- les non ioniques tels que les alcools gras polyéthoxylés, les alkylphénols ou acides gras ou amides d'acides gras polyéthoxylés, les amines grasses polyéthoxylées, les éthanolamides, les dérivés de sucre; ou les oxydes d'amine, et surtout d'alkyldiméthylamine ;
- les cationiques, les chlorures d'alkyldiméthylbenzylammonium ou de dialkyldiméthyl ammonium, les acétates d'imidazoline ou d'alkylamines ou les méthosulfates ;
- les amphotères, les bétaînes (alkyldiméthyl, alkylamidopropyl ou sulfo) ou les dérivés d'imidazoline.

Le procédé tel que décrit ci-dessus, est plus particulièrement destiné au lavage et à la désinfection des locaux d'élevage d'animaux et plus particulièrement à la désinfection des locaux d'élevage avicole ou d'élevages porcins. Il n'est cependant pas limité à cette seule catégorie de local.

L'exemple suivant illustre l'invention sans toutefois la limiter.

### Exemple d'application :

On pulvérise dans un local d'élevage, au canon à mousse de type Karscher muni d'un réservoir de deux litres et capable de délivrer un jet d'eau à un débit à pression atmosphérique de 16 à 25 litres d'eau par minute, une solution moussante désinfectante préparée en diluant 7,5 g d'une solution aqueuse d'acide peracétique contenant :
5,4 % en poids d'acide peracétique,
14,5 % de peroxyde d'hydrogène,
17,9 % d'acide acétique,
0,2 % d'agent tensioactif non ionique (Génapol™ 2908 D),
0,7 % d'inhibiteur de corrosion (dihydrogénophosphate de sodium),
0,1 % d'oxyde d'amine (Aromox™ MCD-W),
0,2 % d'acide hydroxy-éthylidène di phosponique, (DEQUEST™ 2010),
0,4 % d'acide sulfurique et
eau qsp 100 %, et
7,5 g d'AROMOX ™ MCD-W, par litre d'eau.

On asperge ainsi 3 litres de solution désinfectante et moussante par mètre carré de surface traitée. On constate que le bel aspect et bonne tenue de la mousse sur les surfaces désinfectées qui reste intacte près de 30 minutes après la pulvérisation.

On laisse le local sous vide sanitaire, c'est à dire fermé et sans animaux, pendant quelques jours, puis on y thermonébulise 6 ml /m³ de local d'une solution aqueuse préparée par dilution au tiers dans l'eau de ville d'une composition, contenant :
5,4 % en poids d'acide peracétique,
14,5 % de peroxyde d'hydrogène,
17,9 % d'acide acétique,
0,2 % d'agent tensioactif non ionique (Génapol™ 2908 D),
0,7 % d'inhibiteur de corrosion (dihydrogénophosphate de sodium),
0,2 % d'acide hydroxy-éthylidène di phosponique, (DEQUEST™ 2010),
0,4 % d'acide sulfurique et eau qsp 100 %,
au moyen d'un thermonébulisateur **TF 35 EL** commercialisé par la société **IGE-BA.**

On laisse ensuite le local au repos pendant une heure puis on le ventile pendant quinze minutes avant réutilisation.

L'analyse des prélèvements effectués avant la ventilation finale selon la norme française NFT 72281, permet de mettre en évidence un abattement supérieur à 5 log en terme de bactéricidie, supérieur à 4 log en terme de fungicidie et supérieur à 3 log en terme de sporicidie.

On constate que la composition essayée n'a pas d'équivalent connue en termes d'efficacité désinfectante globale par voie aérienne. D'autre part après plusieurs essais, on ne constate aucune détérioration de l'appareil pouvant être préjudiciable aux opérations de thermonébulisation ultérieures.

## Revendications

1. Procédé de nettoyage et de désinfection d'un local, comprenant au moins une étape de désinfection primaire, suivie d'au moins une étape de désinfection secondaire réalisée par thermonébulisation d'une solution aqueuse d'acide peracétique, **caractérisé en ce que** l'étape de désinfection primaire est effectuée par pulvérisation d'un mélange d'une solution aqueuse d'acide peracétique avec au moins un agent moussant..

2. Procédé tel que défini à la revendication 1, dans lequel l'agent tensioactif moussant utilisé, est un composé résistant à l'oxydation par l'acide peracétique et/ou par le peroxyde d'hydrogène.

3. Procédé tel que défini à la revendication 2, dans lequel l'agent tensioactif moussant utilisé est un oxyde d'amine de formule (I) :
(R₁)(R₂)(R₃)N→O (I)
dans laquelle R₁ représente un radical aliphatique linéaire ou ramifié, saturé ou insaturé comprenant de 6 à 30 atomes de carbone et plus particulièrement de 8 à 18 atomes de carbone, et R₂ et R₃, identiques ou différents, représentent indépendamment l'un de l'autre un radical alkyle linéaire ou ramifié, comportant de 1 à 4 atomes de carbone ou un radical hydroxyalkyle, linéaire ou ramifié, comportant de 1 à 4 atomes de carbone.

4. Procédé tel que défini à l'une des revendications 1 à 3, dans lequel la pulvérisation du mélange de la solution aqueuse d'acide peracétique avec l'agent moussant est réalisée au moyen d'un canon à mousse.

5. Procédé tel que défini à l'une des revendications 1 à 4, dans lequel le rapport pondéral acide peracétique / agent moussant dans la composition finale à pulvériser, est compris entre 0,5 et 2.

6. Procédé tel que défini à l'une des revendications 1 à 5, dans lequel on utilise une solution aqueuse d'acide peracétique comprenant :
- entre 1 % et 10% en poids d'acide peracétique,
- entre 5% et 30% et plus particulièrement entre 5% et 25 % en poids, de peroxyde d'hydrogène et,
- entre 5 % et 25 % et plus particulièrement entre 5% et 15% en poids, d'acide acétique,
que l'on dilue dans l'eau à un taux compris entre 0,05 et 0,1 pour donner une solution diluée à laquelle on ajoute un agent moussant en une quantité telle que le rapport pondéral acide peracétique / agent moussant dans la composition finale à pulvériser, soit compris entre 0,75 et 1,5.

7. Procédé tel que défini à la revendication 6, dans lequel on utilise une solution aqueuse d'acide peracétique comprenant :
- entre 3 % et 6 % en poids d'acide peracétique,
- entre 10 % et 20 % en poids de peroxyde d'hydrogène et.
- entre 10 % et 20 % en poids d'acide acétique,
que l'on dilue dans l'eau à un taux égal à 0,075, pour donner une solution diluée à laquelle on ajoute un agent moussant en une quantité telle que le rapport pondéral acide peracétique / agent moussant dans la composition finale à pulvériser soit égal à 1.

8. Procédé tel que défini à l'une des revendications 1 à 7, **caractérisé en ce qu'**il comprend une étape de lavage, préalable à l'étape de désinfection primaire.

9. Procédé tel que défini à la revendication 8, dans lequel l'étape de lavage et l'étape de désinfection primaire constituent une seule étape de lavage et de désinfection effectuée par pulvérisation d'un mélange d'une solution aqueuse d'acide peracétique avec au moins un agent moussant et détergent.

10. Procédé tel que décrit à l'une des revendications 1 à 9 destiné au lavage et à la désinfection des locaux d'élevage d'animaux et plus particulièrement à la désinfection des locaux d'élevage avicole ou porcin.

## Claims

1. Process for the cleaning and disinfection of a location, which comprises at least one primary disinfection step followed by at least one secondary disinfection step carried out by hot-fogging an aqueous peracetic acid solution, **characterized in that** the primary disinfection step is carried out by spraying a mixture of an aqueous peracetic acid solution together with at least one foam former.

2. Process according to Claim 1, in which the foaming surfactant used is a compound which is resistant to oxidation by peracetic acid and/or by hydrogen peroxide.

3. Process according to Claim 2, in which the foaming surfactant used is an amine oxide of the formula (I):
(R₁)(R₂)(R₃)N→O (I)
in which R₁ represents a straight-chain or branched, saturated or unsaturated aliphatic radical having 6 to 30 carbon atoms, more particularly 8 to 18 carbon atoms, and R₂ and R₃ are identical or different and independently of one another represent a straight-chain or branched alkyl radical having 1 to 4 carbon atoms or a straight-chain or branched hydroxyalkyl radical having 1 to 4 carbon atoms.

4. Process according to any of Claims 1 to 3, in which the mixture of the aqueous peracetic acid solution and the foam former is sprayed by means of a foam gun.

5. Process according to any of Claims 1 to 4, in which the weight ratio between peracetic acid and foam former in the final composition to be sprayed is between 0.5 and 2.

6. Process according to any of Claims 1 to 5, in which an aqueous peracetic acid solution comprising:
- between 1% and 10% by weight of peracetic acid,
- between 5% and 30%, more particularly between 5% and 25% by weight, of hydrogen peroxide and
- between 5% and 25%, more particularly between 5% and 15% by weight, of acetic acid
is used, which is diluted in water at a concentration of between 0.05 and 0.1 to give a dilute solution to which a foam former is added in such an amount that the weight ratio of peracetic acid to foam former in the final composition to be sprayed is between 0.75 and 1.5.

7. Process according to Claim 6, in which an aqueous peracetic acid solution comprising:
- between 3% and 6% by weight of peracetic acid,
- between 10% and 20% by weight of hydrogen peroxide and
- between 10% and 20% by weight of acetic acid
is used, which is diluted in water at a concentration of 0.075 to give a dilute solution to which a foam former is added in such an amount that the weight ratio of peracetic acid to foam former in the final composition to be sprayed equals 1.

8. Process according to any of Claims 1 to 7, **characterized in that** it comprises a wash step before the primary disinfection step.

9. Process according to Claim 8, in which the wash step and the primary disinfection step consist of a single wash and disinfection step which is carried out by spraying a mixture of an aqueous peracetic acid solution and at least one foam former and detergent.

10. Process according to any of Claims 1 to 9 for the washing and disinfection of livestock barns, more particularly for the disinfection of bird-rearing barns or pigsties.

## Patentansprüche

1. Verfahren zur Reinigung und Desinfektion eines Orts, das mindestens einen ersten Desinfektionsschritt und im Anschluß daran einen zweiten Desinfektionsschritt, der mittels Heißvernebelung einer wäßrigen Peressigsäurelösung durchgeführt wird, umfaßt, **dadurch gekennzeichnet, daß** der erste Desinfektionsschritt durch Versprühen einer Mischung aus einer wäßrigen Peressigsäurelösung und mit mindestens einem Schaumbildner durchgeführt wird.

2. Verfahren nach Anspruch 1, bei dem das verwendete schäumende Tensid eine Zusammensetzung, die gegenüber Oxidation durch Peressigsäure und/oder Wasserstoffperoxid beständig ist, ist.

3. Verfahren nach Anspruch 2, bei dem das verwendete schäumende Tensid ein Aminoxid der Formel (I):
(R₁) (R₂) (R₃)N→O (I)
in der R₁ einen geradkettigen oder verzweigten, gesättigten oder ungesättigten aliphatischen Rest mit 6 bis 30 Kohlenstoffatomen, insbesondere 8 bis 18 Kohlenstoffatomen, bedeutet, und R₂ und R₃, die gleich oder verschieden sind, unabhängig voneinander einen geradkettigen oder verzweigten Alkylrest mit 1 bis 4 Kohlenstoffatomen oder einen geradkettigen oder verzweigten Hydroxyalkylrest mit 1 bis 4 Kohlenstoffatomen bedeuten, ist.

4. Verfahren nach einem der Ansprüche 1 bis 3, bei dem die Mischung der wäßrigen Peressigsäurelösung mit dem Schaumbildner mit einer Schaumkanone versprüht wird.

5. Verfahren nach einem der Ansprüche 1 bis 4, bei dem das Gewichtsverhältnis von Peressigsäure zu Schaumbildner in der zu versprühenden Endzusammensetzung zwischen 0,5 und 2 beträgt.

6. Verfahren nach einem der Ansprüche 1 bis 5, bei dem eine wäßrige Peressigsäurelösung verwendet wird, die folgendes umfaßt:
- zwischen 1 Gew.-% und 10 Gew.-% Peressigsäure,
- zwischen 5 Gew.-% und 30 Gew.-%, insbesondere zwischen 5 Gew.-% und 25 Gew.-%, Wasserstoffperoxid sowie
- zwischen 5 Gew.-% und 25 Gew.-%, insbesondere zwischen 5 Gew.-% und 15 Gew.-%, Essigsäure,
die man mit Wasser in einem Verhältnis von zwischen 0,05 und 0,1 verdünnt, wodurch man zu einer verdünnten Lösung gelangt, die man mit einem Schaumbildner in solch einer Menge versetzt, daß das Gewichtsverhältnis von Peressigsäure zu Schaumbildner in der zu versprühenden Zusammensetzung zwischen 0,75 und 1,5 beträgt.

7. Verfahren nach Anspruch 6, bei dem eine wäßrige Peressigsäurelösung verwendet wird, die folgendes umfaßt:
- zwischen 3 Gew.-% und 6 Gew.-% Peressigsäure,
- zwischen 10 Gew.-% und 20 Gew.-% Wasserstoffperoxid sowie
- zwischen 10 Gew.-% und 20 Gew.-% Essigsäure,
die man mit Wasser im Verhältnis 0,075 verdünnt, wodurch man zu einer verdünnten Lösung gelangt, die man mit einem Schaumbildner in solch einer Menge versetzt, daß das Gewichtsverhältnis von Peressigsäure zu Schaumbildner in der zu versprühenden Zusammensetzung gleich 1 ist.

8. Verfahren nach einem der Ansprüche 1 bis 7, **dadurch gekennzeichnet, daß** es vor dem ersten Desinfektionsschritt einen Waschschritt umfaßt.

9. Verfahren nach Anspruch 8, bei dem der Waschschritt und der erste Desinfektionsschritt aus einem einzigen Wasch- und Desinfektionsschritt bestehen, der durch Versprühen einer Mischung aus einer wäßrigen Peressigsäurelösung mit mindestens einem Schaumbildner und Tensid durchgeführt wird.

10. Verfahren nach einem der Ansprüche 1 bis 9 zum Waschen und zur Desinfektion von Tierställen, insbesondere zur Desinfektion von Vogelzuchtställen oder Schweineställen.
